Europäisches Patentamt

European Patent Office

Office européen des brevets

(11) Veröffentlichungsnummer: **0 160 253**
**A2**

(12) # EUROPÄISCHE PATENTANMELDUNG

(21) Anmeldenummer: 85104764.7

(22) Anmeldetag: 19.04.85

(51) Int. Cl.⁴: **C 12 N 11/04**
**C 12 N 11/08, C 12 P 19/24**
**C 12 P 19/12**

(30) Priorität: 02.05.84 DE 3416140
28.07.84 DE 3427889

(43) Veröffentlichungstag der Anmeldung:
06.11.85 Patentblatt 85/45

(84) Benannte Vertragsstaaten:
AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Haese, Wilfried, Dr.
Hauweg 20
D-4050 Mönchengladbach-Neuwerk(DE)

(72) Erfinder: Egerer, Peter, Dr.
Claudiusweg 7
D-5600 Wuppertal 1(DE)

(72) Erfinder: Schmidt-Kastner, Günter, Prof.
Falkenberg 59
D-5600 Wuppertal 1(DE)

(72) Erfinder: Perrey, Hermann, Dr.
Auf der Rheinaue 8
D-4150 Krefeld 11(DE)

(54) Immobilisierung von Protaminobacter rubrum und Verwendung des immobilisierten Präparates zur Umwandlung von Sucrose zu Isomaltulose.

(57) Die Erfindung betrifft ein Verfahren zur Immobilisierung von Protaminobacter rubrum durch Einschluß in polymerisierte Verbindungen, das nach diesem Verfahren erhältliche Präparat und dessen Verwendung zur Herstellung von Isomaltulose aus Sucrose.

Das Verfahren besteht darin, daß man wäßringe Lösungen oder Dispersionen des biologischen Materials mit einer wäßrigen Lösung gut wasserlöslicher, höher molekularer polymerisierbarer Verbindungen, die zwei oder mehr polymerisierbare funktionelle Gruppen pro Molekül und ein Molekulargewicht über 400 besitzen, mischt und anschließend polymerisiert.

EP 0 160 253 A2

./...

FIG.1

FIG. 2

BAYER AKTIENGESELLSCHAFT    5090 Leverkusen, Bayerwerk

Konzernverwaltung RP

Patentabteilung    Ad/li-c

Immobilisierung von Protaminobacter rubrum und Verwendung des immobilisierten Präparates zur Umwandlung von Sucrose zu Isomaltulose

Die Erfindung betrifft Verfahren zur Immobilisierung von Protaminobacter rubrum durch Einschluß in polymerisierte Verbindungen, das nach diesen Verfahren erhältliche Präparat und dessen Verwendung zur Herstellung von Isomaltulose aus Sucrose.

Aus den EP-Sen 49 742 und 49 801 ist bekannt, daß Isomaltulose aus Sucrose mittels immobilisierter Zellen von Protaminobacter rubrum oder mittels gereinigter freier oder immobilisierter Sucrose-Mutase aus Protaminobacter-rubrum hergestellt werden kann. Dabei werden a) die Protaminobacter Zellen über ein Flockungsverfahren oder durch Einschluß in Ca-Alginat oder in $\mathcal{K}$-Carrageenan immobilisiert, b) Lösungen des gereinigten Enzyms Sucrose-Mutase zur Immobilisierung in Hohlfasern eingeschlossen, an feste oder lösliche Träger kovalent oder an feste Träger adsorptiv gebunden.

Le A 22 993 -Ausland

Die Wirtschaftlichkeit eines Herstellungsverfahrens für Isomaltulose wird unter anderem auch von den Kosten des hierzu verwendeten Biokatalysator und dessen Langzeitstabilität bestimmt.

Um Aktivitätseinbußen zu vermeiden, ist ein schonendes Immobilisierungsverfahren für Protaminobacter rubrum nötig, welches hohe Aktivitätsausbeuten und hohe Reproduzierbarkeit bietet. Weiterhin ist es von Vorteil, möglichst billige Trägermaterialien zur Immobilisierung zu verwenden.

Einschlußverfahren in $\varkappa$-Carrageenan oder in Ca-Alginat zeichnen sich zwar durch schonende Bedingungen aus, oftmals stehen jedoch die handelsüblichen Preise für diese Stoffe einer Verwendung im technischen Maßstab entgegen. Hinzu kommt, daß die Matrizen aus $\varkappa$-Carrageenan oder Ca-Alginat sehr anfällig gegenüber Änderungen verschiedener Parameter der wäßrigen Reaktionslösung sind, sofern nicht durch zusätzliche Quervernetzung der Trägermatrix z.B. mittels Glutaraldehyd Stabilisierung erfolgte. Quervernetzer wie Glutaraldeyhd sind jedoch häufig für Minderung biologischer Aktivitäten verantwortlich.

Die Herstellung von immobilisierten Zellen durch Flokkung verwendet in der Regel preisgünstige Flockungshilfsmittel, jedoch ist die Aufarbeitung zum lagerfähigen, gekörnten Katalysator vielfach sehr arbeits- und zeitintensiv und verbunden mit hohem technischen Aufwand an

Le A 22 993

Separations-, Trocken-, Mahl- und Klassiervorrichtungen. Diese Vielzahl von mechanischen Operationen führt auch zu Minderung der Gesamtausbeute an Katalysatormaterial von bis zu 30 %.

Es wurde nun ein Verfahren zur Immobilisierung von Protaminobacter rubrum durch Einschluß in polymerisierte Verbindungen gefunden, das dadurch gekennzeichnet ist, daß Protaminobacter rubrum in einer wäßrigen Lösung oder Dispersion mit einer wäßrigen Lösung von gut wasserlöslichen höhermolekularen polymerisierbaren Verbindungen mit zwei oder mehr funktionellen Gruppen pro Molekül und einem Molekulargewicht von über 400 gemischt wird und diese Mischung, gegebenenfalls nachdem diese zuvor in einem inerten flüssigen Medium zu Perlen zerteilt worden ist, polymerisiert wird.

Die aufgrund dieses Verfahrens immobilisierten Zellen können zur Herstellung von Isomaltulose aus Sucrose verwendet werden.

Die Fermentationsbrühe von Protaminobacter rubrum wird dabei, gegebenenfalls ohne Reinigungsschritte, durch Mikrofiltration oder durch kontinuierliche Zentrifugation konzentriert, besonders vorteilhaft um den Faktor 10. In den so erhaltenen Zellschlamm werden die mit einer geringen Menge Puffer vorgelösten gut wasserlöslichen, höhermolekularen, polymerisierbaren Verbindungen eingebracht und homogen verrührt.

Le A 22 993

Durch das Vorlösen der höhermolekularen, polymerisierbaren Verbindungen in einem geringen Volumen wäßrigen Puffers, der ähnlichen pH-Wert, Viskosität, Leitfähigkeit usw. wie das Zellpräparat aufweist, können abrupte Änderungen während des Einschlusses vermieden und so die biologische Aktivitäten der Zellen weitgehend erhalten bleiben. Der mit gut wasserlöslichen polymerisierbaren höhermolekularen Verbindungen versetzte Zellschlamm von Protaminobacter rubrum wird entweder als Film, eventuell auch unter Einarbeitung eines Stützgewebes, z.B. aus Polyamid (Monodur PA 250 N, Verseidag-Industrietextilien GmbH), oder in einer sonstigen geometrischen Form polymerisiert. Um die für Katalysatoren besonders günstige Perlform zu erzielen, ist es nötig, den mit polymerisierbaren Verbindungen versetzten Zellschlamm in einer Inertphase mechanisch zu Perlen zu zerteilen z.B. durch Rühren, wobei der Perldurchmesser durch die Rührerdrehzahl gesteuert werden kann.

Die Polymerisation kann chemisch oder photochemisch durch Bestrahlung mit Hilfe von Radikalstartern oder Photosensibilisatoren erfolgen.

Die so erhaltenen immobilisierten Zellen von Protaminobacter rubrum zeichnen sich durch hohe spezifische Sucrose-Mutase Aktivitäten sowie gute Langzeitstabilität aus und können direkt ohne weitere Verarbeitung oder Formgebung als Biokatalysatoren zur Umsetzung von Sucrose zu Isomaltulose eingesetzt werden.

Die Aufbewahrung der Katalysatorpräparate kann in wäßrigen aber auch nichtwäßrigen, z.B. organischen Medien, erfolgen. Zusätzlich können Zusätze wie Glykol, Polyetherdiole, z.B. Polyethylenglykole, verwendet werden.

Besonders vorteilhaft ist die Verwendung von perlförmigem immobilisiertem Biokatalysator. Die Perlform stellt besonders bei kleinem Perldurchmesser von z.B. 0,2 mm - 2,0 mm, eine ideale Katalysatorform für die Verwendung in kontinuierlichen Säulenreaktoren dar. Weitere Perldurchmesser liegen bei 0,5 mm bis 1,5 mm, allgemein kann der Perldurchmesser jedoch auch 0,05 mm bis 5 mm betragen. Es ist auch möglich, die immobilisierten Protaminobacter Zellen zu trocknen, trocken zu lagern, und nach Quellen in der Sucrose-haltigen Reaktionslösung direkt wiederzuverwenden.

Im folgenden werden die erfindungsgemäß verwendeten polymerisierbaren wasserlöslichen höhermolekularen Verbindungen, die sich zum Einschluß von Protaminobacter rubrum eignen, näher beschrieben.

Durch die Verwendung der erfindungsgemäßen verwendeten höhermolekularen, bevorzugt zwei oder mehr ungesättigte Gruppen enthaltenden, härtbaren, wasserlöslichen Verbindungen läßt sich eine bessere Steuerung und Reproduzierbarkeit der Permeabilitäten erreichen, da sich diese Eigenschaften im wesentlichen durch die höhermolekularen, wasserlöslichen Verbindungen ergeben. Außerdem besteht durch die Verwendung der erfindungsgemäßen Verbindungen kein Toxizitätsproblem. Niedermolekulare

Le A 22 993

toxische Verunreinigungen können außerdem vor dem Mischen mit dem biologischen Material entfernt werden.

Desweiteren wurde gefunden, daß die gut wasserlöslichen Verbindungen zunächst in einer gewissen Menge Wasser, Pufferlösung, Salzlösung und ähnlichem aufgelöst werden können, womit diese Polymerlösung der wäßrigen Lösung oder Dispersion des biologischen Materials hinsichtlich bestimmter Eigenschaften wie pH-Wert, Salzkonzentration, Viskosität u.a. angepaßt werden kann. Anschließend werden beide Lösungen bzw. die wäßrige Polymerlösung und die Zelldispersion gemischt und polymerisiert. Dieses Verfahren besitzt besonders Vorteile für empfindliche Biokatalysatoren, die dadurch keinen abrupten Änderungen des umgebenden Mediums ausgesetzt werden, wodurch eine Aktivitätseinbuße bei der Herstellung der polymerisierbaren Mischung weitgehend vermieden wird.

Ein weiterer Vorteil der vorliegenden Erfindung ist die mit der guten Wasserlöslichkeit einhergehende große Aufnahmekapazität des Polymeren für wäßrige Lösungen und Suspensionen. Es sind Gewichtsverhältnisse von wäßrigem biologischem Material zu festem Polymer zwischen 1:1 und 30:1 möglich, besonders geeignet sind Gewichtsverhältnisse zwischen 4:1 und 20:1.

Durch den möglichen großen Wassergehalt des immobilisierten biologischen Materials vor und nach der Polymerisation befinden sich die Biomaterialien in einer beson-

Le A 22 993

0160253

ders geeigneten, schonenden Umgebung, wodurch die Aktivität stabil gehalten werden kann und wodurch zusätzlich der Substrat- und Produktfluß begünstigt sind. Durch Variation der Struktur und des Molekulargewichts der polymerisierbaren, gut wasserlöslichen, höhermolekularen Verbindungen sind die Eigenschaften des polymeren Netzwerkkes in weitem Maße variationsfähig. Dadurch lassen sich die Vernetzungsdichte, die Permeabilität, das Quellverhalten und weitere Eigenschaften optimieren, um den Anforderungen des biologischen Materials zu entsprechen.

Zur Immobilisierung wurden wasserlösliche, höhermolekulare, polymerisierbare Verbindungen, die zwei oder mehr polymerisierbare funktionelle Gruppen pro Molekül und ein Molekulargewicht von über 400 besitzen, mit insbesondere Molekulargewichten von 1000 bis 20000, insbesondere Verbindungen mit ethylenisch ungesättigten Gruppen, verwendet.

Ihrem Aufbau nach lassen sie sich als Verbindungen beschreiben, die aus der Reaktion von höhermolekularen, hydrophilen Verbindungen mit Verbindungen, die polymerisierbare ethylenisch ungesättigte Gruppen enthalten, entstehen. Beide Reaktionspartner müssen dabei funktionelle Gruppen enthalten, die erlauben, sie chemisch mit- oder untereinander zu verknüpfen bzw. mit Hilfe von di- oder mehrfunktionellen Reagentien mit- oder untereinander zu verknüpfen.

Le A 22 993

Beispielsweise sind solche höhermolekularen, hydrophilen Verbindungen Polyethylenglycole, Ethylenoxid-Propylenoxid-Block- und Mischpolymerisate, alkoxylierte, besonders ethoxylierte zwei- oder mehrwertige Alkohole sowie gut wasserlösliche Polymerisate, z. B. ganz oder teilweise verseifte Polyvinylacetate, gut wasserlösliche Polykondensate wie z. B. Polyester, hergestellt aus obigen höhermolekularen, hydrophilen Verbindungen mit Di- bzw. Polycarbonsäuren und gut wasserlösliche Polyadditionsverbindungen wie z. B. Polyetherpolyurethane, hergestellt aus obigen höhermolekularen, hydrophilen Verbindungen mit Di- bzw. Polyisocyanaten.

Ferner können die in den obigen genannten höhermolekularen, hydrophilen Verbindungen enthaltenen Hydroxylgruppen ganz oder teilweise nach üblichen Verfahren, wie z. B. Umsetzung mit Ammoniak, in Aminogruppen umgewandelt sein.

Als Verbindungen mit polymerisierbaren, ethylenisch ungesättigten Gruppen sind z. B. geeignet: ungesättigte Carbonsäuren, Säurechloride, Dicarbonsäuren, Säureanhydride oder funktionelle Derivate derselben.

Bevorzugt sind Acrylsäure, Methacrylsäure, Crotonsäure, Acrylsäurechlorid, Methacrylsäurechlorid, Itaconsäure, Fumarsäure, Maleinsäure, Maleinsäureanhydrid, Itaconsäureanhydrid, Glycidylacrylat, Glycidylmethacrylat, Hydroxyethylacrylat, Hydroxypropylacrylat, Hydroxyethylmethacrylat, Hydroxypropylmethacrylat, Isocyanatoethylacrylat, Isocyanatoethylmethacrylat, 4-Isocyanato-3-methyl-but-2-ylacrylat.

Le A 22 993

Die Verknüpfung der höhermolekularen, hydrophilen Verbindungen mit den polymerisierbaren, ethylenisch ungesättigten Verbindungen ist somit unterschiedlichster Natur, beispielsweise ergeben sich Ester-, Ether-, Urethan-, Amid-, Amin- und Harnstoffbindungen. Die Herstellung der Verknüpfung kann dabei nach literaturbekannten Verfahren erfolgen, d. h. beispielsweise durch Umsetzung der Hydroxyl- oder Aminogruppen mit Säuren, Säurehalogeniden oder Säureanhydriden zu den entsprechenden Estern oder Amiden, mit Epoxiden zu den entsprechenden Ethern oder Aminen oder mit Isocyanaten zu den entsprechenden Urethanen oder Harnstoffen.

Ferner kann neben der direkten Verknüpfung der höhermolekularen, hydrophilen Verbindungen mit den Verbindungen, die die ethylenisch ungesättigten Gruppen enthalten, die Verknüpfung auch mittels bi- oder mehrfunktioneller Reagentien erfolgen.

Beispiele solcher bi- oder mehrfunktioneller Reagentien sind di- oder mehrfunktionelle Isocyanate wie Isophorondiisocyanat, Toluylendiisocyanat, Hexamethylendiisocyanat, Biuretgruppenhaltige Polyisocyanate (z. B. Desmodur N, Umsetzungsprodukt von Hexamethylendiisocyanat mit Wasser), Polyisocyanaten, die aus der Umsetzung von Diisocyanaten mit mehrwertigen Alkoholen entstehen (z. B. Desmodur L, Umsetzungsprodukt von Toluylendiisocyanat mit Trimethylolpropan) sowie Di- oder Polyepoxide wie z. B. Bisphenol-A-digylcidylether oder Hexahydrophthalsäurediglycidylester. Die Verknüpfung dieser Reagentien erfolgt dabei z. B. mit den Hydroxylgruppen

Le A 22 993

der höhermolekularen, hydrophilen Verbindungen und hydroxylgruppenhaltigen Verbindungen, die polymerisierbare, ethylenisch ungesättigte Gruppen enthalten, unter Urethan- bzw. Etherbildung.

Zur Herstellung der erfindungsgemäßen Verbindungen können die verschiedenen Verknüpfungsprinzipien auch untereinander kombiniert werden.

Art und Anteil der hydrophilen, höhermolekularen Verbindungen, der Verbindungen, die die ethylenisch ungesättigten Gruppen tragen sowie gegebenenfalls der di- oder mehrfunktionellen Reagentien müssen jedoch so gewählt werden, daß die daraus hergestellten erfindungsgemäßen, höhermolekularen, polymerisierbaren Verbindungen so hydrophil sind, daß die gute Wasserlöslichkeit und große Aufnahmekapazität für wäßrige Lösungen oder Dispersionen an biologischem Material gegeben ist.

Als bevorzugte wasserlösliche, höhermolekulare, polymerisierbare Verbindungen seien genannt:
- Verbindungen, die aus Polyetherpolyolen, deren Hydroxylgruppen teilweise mit ungesättigten Carbonsäuren verestert und zum anderen Teil mit isocyanatgruppenhaltigen Derivaten ungesättigter Carbonsäuren umgesetzt sind, hergestellt sind.

Besonders bevorzugt seien genannt:
- Verbindungen, die aus Polyethylenglykolen mit einem Molekulargewicht größer 400, deren Hydroxylgruppen

Le A 22 993

teilweise mit Acrylsäure oder Methacrylsäure verestert und zum anderen Teil mit Isocyanatoethylmethacrylat oder 4-Isocyanato-3-methyl-but-2-ylacrylat umgesetzt sind, hergestellt sind.

Bei der Herstellung können die Hydroxylgruppen der Polyetherpolyole zunächst teilweise mit den isocyanatgruppenhaltigen Derivaten ungesättigter Carbonsäuren umgesetzt werden und anschließend zum anderen Teil mit den ungesättigten Carbonsäuren verestert werden, bevorzugt ist jedoch, zuerst die teilweise Umsetzung mit den ungesättigten Carbonsäuren und anschließend die Umsetzung des anderen Teils der Hydroxylgruppen mit den isocyanatgruppenhaltigen Derivaten ungesättigter Carbonsäuren.

Weiterhin seien als bevorzugte, wasserlösliche, höhermolekulare, polymerisierbare Verbindungen genannt:
- Verbindungen, die aus Polyetherpolyolen, deren Hydroxylgruppen teilweise mit ungesättigten Carbonsäuren verestert und zum anderen Teil mit di- oder mehrfunktionellen Isocyanaten umgesetzt sind, hergestellt sind.

Besonders bevorzugt hierbei seien genannt:
- Verbindungen, die aus Polyethylenglykolen mit einem Molekulargewicht größer 400, deren Hydroxylgruppen teilweise mit Acrylsäure oder Methacrylsäure verestert und zum anderen Teil mit Isophorondiisocyanat, Toluylendiisocyanat, biuretgruppenhaltigen Polyisocyanaten oder Polyisocyanaten, die aus der Umsetzung von Diisocyanaten mit mehrwertigen Alkoholen entstehen, hergestellt sind.

Le A 22 993

0160253

Bei der Herstellung können die Hydroxylgruppen der Polyetherpolyole zunächst teilweise mit den di- oder mehrfunktionellen Isocyanaten umgesetzt werden und anschließend zum anderen Teil mit ungesättigten Carbonsäuren verestert werden.

Vorteilhafter ist es jedoch, zuerst die teilweise Veresterung und anschließend die Umsetzung mit den di- oder
mehrfunktionellen Isocyanaten durchzuführen.

Die Polymerisation kann unter Inertgas durchgeführt und
in Gegenwart üblicher Radikalstarter wie Azo-bis(isobutyronitril), t-Butylperoctoat, Benzoylperoxid, Dicyclohexylperoxydicarbonat, Methylethylketonperoxid, Cumolhydroperoxid, Acetyl-cyclohexansulfonyl-peroxid, Dicumylperoxid,
Kaliumperoxodisulfat oder Ammoniumperoxodisulfat sowie
durch Redoxsysteme wie Kaliumperoxodisulfat-Riboflavin,
Kaliumperoxodisulfat-Natriumbisulfit, Wasserstoffperoxid-
Verbindungen des zweiwertigen Eisens erfolgen. Als Beschleuniger können ebenfalls zahlreiche Verbindungen
dienen, z.B. N,N,N',N'-Tetramethylethylendiamin oder
ß-Dimethylaminopropionitril.

Eine weitere Möglichkeit der Polymerisation besteht
in der Bestrahlung der Mischung mit aktinischem Licht.
Es eignen sich beispielsweise Hochdruckquecksilberlampen, Niederdruckquecksilberlampen, Fluoreszenzlampen, Xenonlampen, Kohlelichtbögen sowie Sonnenbestrahlung. Eine Bestrahlung mit Elektronenstrahlen oder
Gammastrahlen ist ebenfalls möglich, jedoch muß mit
einer gewissen Schädigung des biologischen Materials

Le A 22 993

gerechnet werden. Ferner kann die Photopolymerisation durch Photosensibilisatoren beschleunigt werden. Es können bekannte Photosensibilisatoren wie $\alpha$-Carbonylalkohole, z.B. Benzoin oder Acetoin, Acyloinether wie Benzoinmethylether, Benzoinethylether, Benzoinisopropylether, $\alpha$-substituierte Acyloine wie $\alpha$-Methylbenzoin und $\alpha$-Methoxybenzoin u.a. sowie durch ionische Gruppen wie z.B. Carbonsäure-, Sulfonsäure- oder Aminogruppen modifizierte und dadurch wasserlöslich gemachte Derivate verwendet werden.

Desweiteren können polycyclische aromatische Verbindungen wie Naphthol und Hydroxyanthracen, Azoamide wie beispielsweise 2-Cyano-2-butylazoformamid sowie Metallsalze wie Uranylnitrat und Eisenchlorid, wie auch Mercaptane, Disulfide, Halogenide oder Farbstoffe verwendet werden.

Das immobilisierte Biomaterial kann während der Polymerisation durch Formgebung nahezu beliebige Gestalt annehmen. Es sind Folien, Filme, Formteile usw. darstellbar. Weiterhin können durch Einbau von Stützgeweben die mechanischen Eigenschaften modifiziert werden. Ebenso sind Beschichtungen von Elektroden, Membranen, oder anderen Materialien durchführbar.

Desweiteren kann die Polymerisation in Form einer Dispersion des noch nicht polymerisierten biologischen Materials in einem inerten flüssigen Medium wie z.B. aliphatischen, cycloaliphatischen und aromatischen Kohlenwasserstoffen, sowie halogenierten Derivaten derselben, des-

Le A 22 993

weiteren in Ethern, Estern oder sonstigen, nicht wassermischbaren Stoffen wie auch in Siliconölen und Paraffinölen, bzw. auch Gemischen derselben untereinander, durchgeführt werden, wobei in Abhängigkeit von der Verteilung, die durch Rühren, Einleiten von Inertgas, z.B. Stickstoff, oder sonstiges Zudosieren der wäßrigen Phase, z.B. durch Einpumpen oder Einsprühen in die inerte Phase erreicht werden kann, die Größe der Perlen sehr variabel ist. Weiterhin kann durch Zusätze zur Wasser- oder Inertphase deren Viskosität, Dichte, Oberflächenspannung, usw. verändert werden, was ebenfalls den Perldurchmesser beeinflußt.

Durch die hohe Aufnahmekapazität für Wasser - besonders gut eignen sich Gewichtsverhältnisse von wäßriger Lösung oder Suspension des biologischen Materials zu festem Polymer zwischen 4:1 und 20:1 - die das immobilisierte Material vor und nach der Polymerisation besitzt, ergeben auch Vorteile hinsichtlich der Wirtschaftlichkeit des Verfahrens. Dadurch können einerseits große Mengen an wäßrigen Lösungen oder Dispersionen des biologischen Materials in einer relativ kleinen Menge der polymeri-sierbaren Verbindungen eingeschlossen werden, anderer-seits können dadurch Fermenterbrühen direkt oder nach Konzentrierung z.B. durch Mikrofiltration oder Zentrifugieren immobilisiert werden, ohne daß weitere Bestandteile des Fermentermediums entfernt werden müssen.

Desweiteren ist es jedoch vor und nach der Immobilisierung auch möglich, geringere Mengen der wäßrigen Lösungen zu verwenden, sowie einen Teil des Anteils an Wasser gegen andere Flüssigkeiten wie z.B. Alkohole zu ersetzen.

Le A 22 993

Es ist auch möglich, die immobilisierten Protaminobacter rubrum Zellen in anderen als wäßrigen Lösungen, z.B. in aliphatischen oder aromatischen Kohlenwasserstoffen, einzusetzen.

Die nach vorstehendem Verfahren immobilisierten Protaminobacter rubrum Zellen lassen sich als Biokatalysatoren zu Biotransformationen einsetzen.

Le A 22 993

## Beispiele

## Fermentation von Protaminobacter rubrum

Zur Produktion von Sucrose Mutase wurde der Stamm Protaminobacter rubrum (CBS 574.77) verwendet.

Die Nährlösung besteht aus 5 % Dicksaft, 2 % Maisquellwasser und 0,05 % $(NH_4)_2 HPO_4$, der pH-Wert beträgt 7.1. Mit 1 ml einer Protaminobacter rubrum Abschwemmung wurden in einem 1 l Erlenmeyerkolben 200 ml Nährlösung beimpft. Die Fermentation läuft 15 h bei 31°C auf der Rundschüttelmaschine.

Mit dieser Vorkultur wurde ein 300 l Fermenter mit 200 l obiger Nährlösung angeimpft und 16 h fermentiert.

Die Aktivität der Fermenterlösung an Sucrose Mutase betrug 9.1 U/ml. 1 U = 1 µMol Sucrose/min umgesetzt.

Die 200 l Fermenterlösung wurden durch kontinuierliche Zentrifugation etwa um den Faktor 10 konzentriert, und diese konzentrierte Protaminobacter Zellsuspension direkt zu nachfolgenden Immobilisierungsexperimenten im Pilot-Maßstab eingesetzt.

Le A 22 993

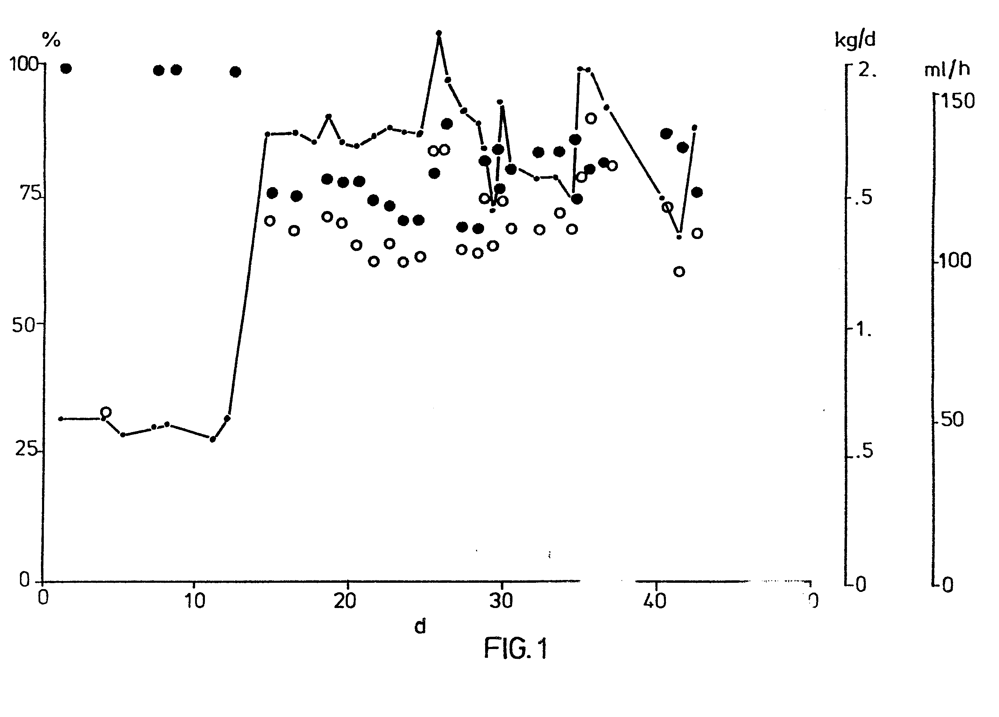

Beispiel A (s. Fig. 1)

2189 g Polyethylenglykol mit einem mittleren Molekulargewicht von 1550 (Chemische Werke Hüls) wurden mit 98,4 g
Acrylsäure unter Zusatz von 22,8 g p-Toluolsulfonsäure,
52,3 g Di-tert.-butylhydrochinon sowie 2,3 g p-Methoxyphenol in Toluol verestert.

1100 g des Esters wurden mit 74,2 g Isophorondiisocyanat
unter Zusatz von 0,15 g Desmorapid S0 umgesetzt.

100 g dieses so erhaltenen polymerisierbaren Acrylatharzes wurden mit 1 g Irgacure (Ciba-Geigy) gemischt und
mit 40 g (0,01 M) Phosphatpuffer pH 7 versetzt, sodaß
eine Lösung entstand.

Diese Lösung wurde mit 348 g der durch Mikrofiltration
konzentrierten Fermenterbrühe von Protaminobacter rubrum
gemischt und als 500 µm dicke Filme (Format 40 cm x 60
cm) mittels vier Quecksilberhochdrucklampen polymerisiert.

Anschließend wurden die Filme in kleine Stücke zerschnitten (ungefähr 0,5 cm$^2$) und in eine thermostatisierbare 1 l Säule eingebracht. Der Bioreaktor wurde
bei 30°C mit einer 50 % Sucroselösung nach Sterilfilter (Millistak, Fa. Millipore) durchströmt. Nach Einstellen eines Durchflusses von etwa 130 ml/h wurde ein
Sucroseumsatz von etwa 70-80 % erzielt. Die Produktlösung wurde per HPLC analysiert. Nach 40 Tagen war noch
keine Aktivitätsminderung festzustellen.

Le A 22 993

In Fig. 1 wird die kontinuierliche Umwandlung von Saccharose in Palatinose mit eingeschlossenen Zellen von Protaminobacter rubrum dargestellt:

1 l Reaktorvolumen, 50 % Saccharose, 30°C.

.-.-.    Durchfluß $/\overline{ml/h}/$
●●      % umgesetzte Saccharose;
○○      Palatinose $/\overline{kg/d}/$

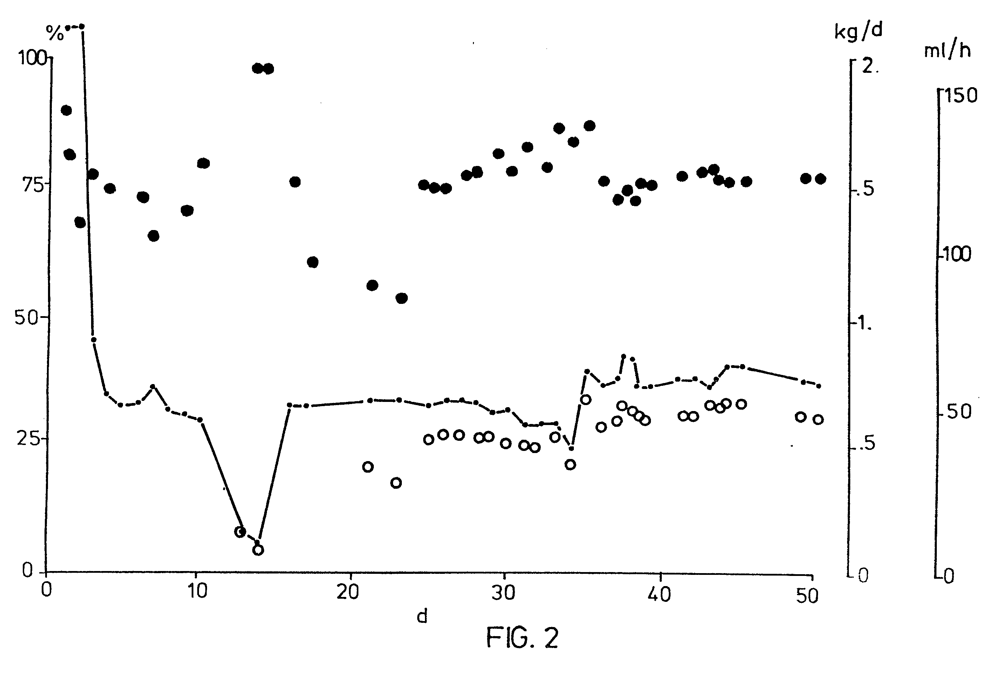

Beispiel B (s. Fig. 2)

Die Protaminobacter rubrum Zellen enthaltende Mischung aus Beispiel A (175 g) wurde in Polyamidgewebestücke (Monodur 250 N, Verseidag-Industrietextilien GmbH) des Formats 40 cm x 60 cm verstrichen und durch Belichtung polymerisiert. Die so hergestellten verstärkten Filme wurden gemeinsam mit unbeschichtetem Polyamidgewebe so aufgewickelt, daß sich jeweils zwischen 2 Lagen des Films eine Lage des unbeschichteten Gewebes befand. Das zylindrisch aufgewickelten Material wurde in eine 1 l Säule hineingeschoben, wobei der Säuleninnendurchmesser so gewählt wurde, daß das Material den ganzen Säulendurchmesser ausfüllte, und bei 30°C mit einer 50 % Sucroselösung, pH 6,5, durchströmt. Die Sucrose-Vorratslösung wurde über ein Sterilfilter Milli-Stak (Fa. Millipore) per Schlauchpumpe durch die Reaktorsäule gepumpt. Nach Einstellen eines Durchflusses von etwa 60 ml/h wurde ein konstanter Sucroseumsatz von etwa 75 % erzielt. Die Produktlösung wurde per HPLC analysiert und wies 68,5 % Isomaltulose 4,5 % 1,1'-Disaccharid, 1,2 % Fructose 0,8 % Glucose auf.

Le A 22 993

Die Sucrose Mutase-Aktivität der immobilisierten Protaminobacter-Zellen war über 50 Tage hinweg stabil.

In Fig. 2 wird die kontinuierliche Umwandlung von Saccharose in Palatinose mit eingeschlossenen Protaminobacter
rubrum Zellen dargestellt.

1 l Reaktorvolumen, 50 % Saccharose, 30°C.
.-.-.  Durchfluß $\sqrt{\overline{ml/\underline{h}}}$
●●  % umgesetzte Saccharose;
○○  Palatinose $\sqrt{\overline{kg/\underline{d}}}$

Beispiel C

1000 g Polyethylenglykol mit einem mittleren Molekulargewicht von 1000 (Chemische Werke Hüls) wurde mit 72 g
Acrylsäure unter Zusatz von 9,2 g p-Toluolsulfonsäure
und 1,0 g Di-tert.-butylhydrochinon sowie 1,0 g p-
Methoxyphenol in Toluol verestert.

750 g des so erhaltenen Esters wurde mit 83,3 g Isophorondiisocyanat unter Zusatz von 0,09 g Desmorapid
SO umgesetzt.

Aus 500 g des so erhaltenen polymerisierbaren Acrylatharzes, 5 g  Irgacure (Ciba-Geigy), 100 g Phosphatpuffer,
0,01 M, pH 7, sowie 2750 g konzentrierter Fermenterbrühe wurden analog Beispiel A kleine Filmstücke hergestellt, mit denen eine 5 l Säule gefüllt wurde.

Le A 22 993

Dieser Bioreaktor wurde bei 30 °C nach Vorschalten eines Millistak-Sterilfilters (Fa. Millipore) mit 50 % Sucroselösung, pH 7,0, durchströmt. Nach Einstellung eines Durchflusses von etwa 500 ml/h wurde ein Sucroseumsatz zwischen 78,7 und 80,0 % gemessen.

Per HPLC ergab sich eine Produktverteilung von beispielsweise 21,25 % Sucrose, 69,91 Isomaltulose, 5,81 % 1,1'-Disaccharid, 1,84 % Fructose, 1,19 % Glucose. Über 40 Tage hinweg wurde keine Aktivitätsminderung des Biokatalysators festgestellt.

Beispiel D

1658 g des in Beispiel A hergestellten Esters wurden mit 182 g Isocyanatoethylmethacrylat (DOW Chemicals) unter Zusatz von 0,36 g Desmorapid SO umgesetzt.

700 g dieses so erhaltenen polymerisierbaren Acrylatharzes wurden mit 210 g Phosphatpuffer, 0,01 M, pH 7,0 und 35 g 1,2-Diphenyl-2-hydroxy-3-/N(N-Methyl)-pyrrolidinium7-propan-1-on-methylsulfat gemischt. Zu dieser Lösung wurden 4,9 kg der durch Mikrofiltration konzentierten Fermenterbrühe, die Protaminobacter rubrum Zellen enthält, gegeben und diese Mischung in 32,2 kg eines Paraffinöl-Siliconöl-Gemisches mit einer spez. Dichte von 0,9 g /cm$^3$ getropft. Nach Zusatz von 5 g Emulgator (Span 80) wurde durch intensives Rühren und durch Stickstoffeinleitung eine Zerteilung der obigen zellenthaltenden Mischung in Perlen erreicht, die mittels 4 Quecksilberhochdrucklampen und 1 1/2 stündiger Bestrahlung polymerisiert wurden.

Le A 22 993

Die erhaltenen Perlen (mittlerer Durchmesser 1 mm) wurden in eine 10 1 Säule gefüllt und bei 30°C mit einer 50 % Sucroselösung nach Sterilfiltration durch Millistak-Filter (Fa. Millipore) durchströmt. Der pH-Wert wurde zwischen 6,4 und 6,6 eingestellt. Schon nach kurzer Zeit wurde ein relativ konstanter Durchfluß von etwa 2,8 1/h erreicht und die Produktlösung per HPLC analysiert. Ein typisches Produktspektrum sah wie folgt aus: 2,53 % Fructose, 1,86 % Glucose, 15.11 % Sucrose, 74,42 % Isomaltulose, 6,08 % 1,1'-Disaccharid. Es waren keine Tri-, Tetra- oder sonstige Oligosaccharide nachweisbar.

Beispiel E

1200 g des Esters aus Beispiel A wurden mit 63,3 g Desmodur N und 38,3 g Isophorondiisocyanat unter Zusatz von 0,2 g Desmorapid SO umgesetzt.

Aus 800 g dieses so erhaltenen polymerisierbaren Acrylatharzes, 40 g Irgacure (Ciba-Geigy), 320 g Phosphatpuffer, 0,01 M, pH 7,0, sowie 4,0 kg Protaminobacter Zellsuspension, konzentriert um den Faktor 10, und 30 kg Siliconöl mit einer Dichte von 0,95 g/cm$^3$ wurden analog Beispiel D Perlen (mittlerer Durchmesser 1,5 mm) hergestellt und in eine 10 1 Säule gefüllt.

Der mit immobilisierten Protaminobacter rubrum gefüllte 10 1 Bioreaktor wurde bei 30°C von einer sterilfiltrierten 50 % Sucroselösung durchströmt und nach Einstellen eines Durchflusses von etwa 3,05 1/h kontinuierlich betrieben. Der pH-Wert wurde dabei auf 6,4-6,6 geregelt.

Le A 22 993

Der Reaktor wurde über 2 Monate hinweg ohne nennenswerte
Aktivitätsverluste betrieben. Ein typisches Analysenbeispiel, welches per HPLC ermittelt wurde:

31,1 % Sucrose, 60,7 % Isomaltulose, 1,7 % Fructose,
1,4 % Glucose, 5,1 % 1,1'-Disaccharid, keine Oligosaccharide.

Die Durchflußrate des Bioreaktors wurde nun auf etwa
1,75 l/h erniedrigt und folgendes Produktspektrum ermittelt: 4,3 % Fructose, 3,9 % Glucose, 0,9 % Sucrose,
81,7 % Isomaltulose, 9,2 % 1,1'-Disaccharid, keine
Oligosaccharide.

**Beispiel F**

55.5 g des in Beispiel A verwendeten polymerisierbaren
Harzes wurden mit 12 g Phosphatpuffer (0.01 M, pH 7.0)
versetzt, sodaß eine Lösung entstand.

Diese Lösung wurde mit 500 g der durch Cross-Flow Mikrofiltration konzentrierten Fermenterbrühe von Protaminobacter rubrum gemischt und darin 1.1 g Ammoniumperoxodisulfat gelöst. Nach Zugabe von 1.1 g N,N,N',N',-Tetramethylethylendiamin wurde die Mischung intensiv gerührt und in eine Schale ausgegossen, sodaß die Schichtdicke ungefähr 3 mm betrug. Die Polymerisation setzte
innerhalb weniger Minuten ein. Das polymerisierte Produkt wurde in kleine Stücke der Größe 5 x 5 x 3 mm zerschnitten und in eine 1 L-Säule gepackt.

Le A 22 993

Im Dauerbetrieb bei 30°C, 50 % Sucroselösung, stellte sich bei einem Durchfluß von 225 ml/h ein Umsatz von 58 % der angebotenen Sucrose ein. Nach 8 Tagen war noch keine Abnahme der Produktivität festzustellen.

<u>Le A</u> 22 993

Patentansprüche

1.  Verfahren zur Immobilisierung von Protaminobacter rubrum durch Einschluß in polymerisierte Verbindungen, dadurch gekennzeichnet, daß wäßrige Lösungen oder Dispersionen des biologischen Materials mit einer wäßrigen Lösung gut wasserlöslicher, höhermolekularer polymerisierbarer Verbindungen, die zwei oder mehr polymerisierbare funktionelle Gruppen pro Molekül und ein Molekulargewicht über 400 besitzen, gemischt und anschließend polymerisiert werden.

2.  Verfahren nach Anspruch 1, dadurch gekennzeichnet, daß ein radikalischer Polymerisationsinitiator und/oder ein radikalisches Polymerisationsinitiatorsystem und/oder ein Beschleuniger zugesetzt wird.

3.  Verfahren nach Anspruch 2, dadurch gekennzeichnet, daß ein wasserlöslicher radikalischer Polymerisationsinitiator, insbesondere Ammoniumperoxodisulfat und ein wasserlöslicher Beschleuniger wie N,N,N',N'-Tetramethylethylendiamin zugesetzt wird.

4.  Verfahren nach Anspruch 1, dadurch gekennzeichnet, daß ein Photosensibilisator zugesetzt wird und die Polymerisation durch Bestrahlung erfolgt.

5.  Verfahren nach einem der vorhergehenden Ansprüche, dadurch gekennzeichnet, daß das Gewichtsverhältnis der wäßrigen Lösung oder der Dispersion von Protaminobacter rubrum zu der gut wasserlöslichen poly-

Le A 22 993

merisierbaren höhermolekularen Verbindung bzw. deren wäßrigen Lösungen zwischen 1:1 bis 30:1, insbesondere zwischen 4:1 bis 20:1 liegt.

6. Verfahren nach einem der vorgehenden Ansprüche, dadurch gekennzeichnet, daß die gut wasserlöslichen, höhermolekularen, polymerisierbaren Verbindungen aus Polyetherpolyolen, deren Hydroxylgruppen teilweise mit ungesättigten Carbonsäuren verestert und zum anderen Teil mit isocyanatgruppenhaltigen Derivaten ungesättigter Carbonsäuren umgesetzt sind, hergestellt sind.

7. Verfahren nach Anspruch 6, dadurch gekennzeichnet, daß

a) die Polyetherpolyole Polyethylenglykole mit einem MW von 400 und größer sind,

b) die ungesättigten Carbonsäuren Acrylsäure und/ oder Methacrylsäure sind,

c) die isocyanatgruppenhaltigen Derivate ungesättigter Carbonsäuren Isocyanatoethylacrylat, Isocyanatoethylmethacrylat und/oder 4-Isocyanato-3-methyl-but-2-ylacrylat sind.

8. Verfahren nach Anspruch 1 bis 5, dadurch gekennzeichnet, daß die gut wasserlöslichen, höhermolekularen, polymerisierbaren Verbindungen aus Polyetherpolyolen, deren Hydroxylgruppen teilweise mit ungesättigten Carbonsäuren verestert und zum anderen Teil mit di- oder mehrfunktionellen Isocyanaten umgesetzt sind, hergestellt sind.

Le A 22 993

9.  Verfahren nach Anspruch 8, dadurch gekennzeichnet, daß

a) die Polyetherpolyole Polyethylenglykole mit einem MW von 400 und größer sind,

b) die ungesättigten Carbonsäuren Acrylsäure und/ oder Methacrylsäure sind,

c) die di- oder mehrfunktionellen Isocyanate Isophorondiisocyanat, Toluylendiisocyanat, Hexamethylendiisocyanat, biuretgruppenhaltige Polyisocyanate und/oder Polyisocyanate, die aus der Umsetzung von Diisocyanaten mit mehrwertigen Alkoholen entstehen, sind.

10. Verfahren nach vorhergehenden Ansprüchen, dadurch gekennzeichnet, daß zur Immobilisierung direkt Fermentationsbrühen, gegebenenfalls nach vorhergehender Konzentrierung, verwendet werden ohne Entfernen von Bestandteilen des Fermentationsmediums.

11. Verfahren nach einem der vorhergehenden Ansprüche, dadurch gekennzeichnet, daß Zellen oder Enzyme in Gegenwart von Desinfektionsmitteln, Bakteriziden oder Fungiziden immobilisiert werden, somit chemisch sterilisiert in den Bioreaktor überführt werden und die Sterilisationsmittel vor Anlauf der Reaktion aus dem geschlossenen, sterilen Reaktorsystem ausgewaschen werden.

12. Verfahren nach einem der vorhergehenden Ansprüche, dadurch gekennzeichnet, daß eine Zellsuspension von Protaminobacter rubrum, bevorzugt jene mit einem

Le A 22 993

Feststoffgehalt von 5 % bis 25 %, mit einer wäßrigen Lösung der gut wasserlöslichen höhermolekularen, polymerisierbaren Verbindung gemischt und polymerisiert wird.

13. Verfahren nach Anspruch 12, dadurch gekennzeichnet, daß das Gewichtsverhältnis von wäßriger Zellsuspension zu festem Polymer von 1:1 bis 30:1, bevorzugt von 4:1 bis 20:1 beträgt.

14. Verfahren nach einem der vorhergehenden Ansprüche, dadurch gekennzeichnet, daß die Protaminobacter rubrum enthaltende wäßrige Lösung der gut wasserlöslichen, höhermolekularen, polymerisierbaren Verbindung in einer zweiten, nichtwäßrigen Inertphase zu Perlen dispergiert und diese perlförmige Wasserphase polymerisiert wird.

15. Verfahren nach einem der vorhergehenden Ansprüche, dadurch gekennzeichnet, daß

- das Gewichtsverhältnis Wasserphase zu Inertphase zwischen 1:1 und 1:20 liegt,

- die Zerteilung der wäßrigen Phase zu Perlen durch Rühren und/oder Durchleiten von Inertgas, oder durch Einsprühen oder Einpumpen der wäßrigen Phase in die Inertphase erfolgt,

- Perlen der mittleren Durchmesser 0,05 mm bis 5 mm, bevorzugt 0,2 mm bis 2 mm, besonders

Le A 22 993

bevorzugt 0,5 mm bis 1,5 mm demgemäß hergestellt werden,

- die Inertphase einen mit Wasser schlecht oder nicht mischbaren Stoff darstellt, wie beispielsweise aliphatische, cycloaliphatische oder aromatische Kohlenwasserstoffe, deren Derivate sowie Siliconöle und Paraffinöle bzw. auch Gemische derselben untereinander.

16. Immobilisierte Protaminobacter rubrum Zellen erhältlich nach einem der vorhergehenden Ansprüche.

17. Verwendung der immobilisierten Protaminobacter rubrum Zellen nach einem der vorhergehenden Ansprüche zu Biotransformationen.

18. Verwendung des nach den vorhergehenden Ansprüchen hergestellten immobiliserten Protaminobacter rubrum Zellen enthaltenden Materials zur Umwandlung von Sucrose zu Isomaltulose.

FIG. 1

0160253

FIG. 2